(19) Europäisches Patentamt
European Patent Office
Office européen des brevets

(11) **EP 0 813 600 B1**

(12) **EUROPÄISCHE PATENTSCHRIFT**

(45) Veröffentlichungstag und Bekanntmachung des Hinweises auf die Patenterteilung:
**18.05.2005 Patentblatt 2005/20**

(51) Int Cl.[7]: **C12N 15/54**, C12P 19/30, C12N 9/90, C12Q 1/48, C12N 9/12

(21) Anmeldenummer: **96904730.7**

(22) Anmeldetag: **01.03.1996**

(86) Internationale Anmeldenummer:
**PCT/DE1996/000371**

(87) Internationale Veröffentlichungsnummer:
**WO 1996/027670 (12.09.1996 Gazette 1996/41)**

(54) **PHOTOMETRISCHER TEST ZUR BESTIMMUNG VON PYROPHOSPHAT NACH DESSEN ENZYMATISCHER UMSETZUNG**

PHOTOMETRICAL TEST TO DETECT PYROPHOSPHATE AFTER ITS CONVERTION BY ENZYMES

TEST PHOTOMETRIQUE POUR DETECTER LE PYROPHOSPATE APRES SA CONVERSION ENZYMATIQUE

(84) Benannte Vertragsstaaten:
**AT BE CH DE DK ES FI FR GB IE IT LI LU NL PT SE**

(30) Priorität: **03.03.1995 DE 19507449**
**15.05.1995 DE 19517093**
**23.02.1996 DE 19606651**

(43) Veröffentlichungstag der Anmeldung:
**29.12.1997 Patentblatt 1997/52**

(73) Patentinhaber: **Forschungszentrum Jülich GmbH 52425 Jülich (DE)**

(72) Erfinder:
• **RITTER, Jörg, Eberhard**
**D-52382 Niederzier (DE)**
• **ELLING, Lothar**
**D-52066 Aachen (DE)**
• **KULA, Maria-Regina**
**D-52382 Niederzier (DE)**
• **VERSECK, Stefan**
**D-42103 Wuppertal (DE)**

(74) Vertreter: **Pielken, Petra, Dr.**
**Becker-Gundahl-Str. 36**
**81479 München (DE)**

(56) Entgegenhaltungen:
**WO-A-87/05937 DE-A- 3 626 915**

• **JOURNAL OF BIOLOGICAL CHEMISTRY, Bd. 239, Nr. 10, 1964, MD US, Seiten 3119-3129, XP000574872 J. PREISS AND E. WOOD: "Sugar nucleotide reactions in Arthrobacter" in der Anmeldung erwähnt**
• **METHODS IN ENZYMOLOGY, Bd. v, 1962, Seiten 171-174, XP000574873 A. MUNCH-PETERSEN: "GDPM pyrophosphorylase" in der Anmeldung erwähnt**
• **MOLECULAR MICROBIOLOGY, Bd. 5, Nr. 3, 1991, Seiten 695-713, XP000574087 X. JIANG ET AL: "Structure and sequence of the rfb (O antigen) gene cluster of Salmonella serovar typhimurium (strain LT2)" in der Anmeldung erwähnt**
• **J. BACTERIOL. (1994), 176(11), 3126-39 CODEN: JOBAAY;ISSN: 0021-9193, 1994, XP002007414 JAYARATNE, PADMAN ET AL: "Cloning and analysis of duplicated rfbM and rfbK genes involved in the formation of GDP-mannose in Escherichia coli O9:K30 and participation of rfb genes in the synthesis of the group I K30 capsular polysaccharide"**
• **JOURNAL OF BIOTECHNOLOGY 34 (2). 1994. 157-163. ISSN: 0168-1656, XP002007415 ELLING L ET AL: "Purification of UDP-glucose pyrophosphorylase from germinated barley (malt)."**

- **JOURNAL OF BIOLOGICAL CHEMISTRY, Bd. 250, Nr. 22, 1975, MD US, Seiten 8690-8695, XP000601574 W. O'BRIEN ET AL: "Isolation and characterization of a pyrophosphate-dependent phosphofructokinase from Propionibacterium shermanii" in der Anmeldung erwähnt**

**Beschreibung**

Die Erfindung betrifft einen photometrischen Test zur Bestimmung des durch ein Pyrophosphat freisetzendes Enzym entstehendes Pyrophosphat.

Aus dem Artikel "Isolation and Charakterization of a Pyrophosphat-dependent Phosphofructokinase from Propionibacterium sherman II" von William E. O'Brien et.al. im Journal of Biological Chemistry, Nov. 25, 1975 Vol 250 Nr. 22 ist eine pyrophosphatabhängige phosphofructokinase bekannt.

Es ist die Aufgabe der Erfindung, einen Test zur Verfügung zu stellen, mit dem das durch ein pyrophosphat freisetzendes Enzym freigesetzte Pyrophosphat bestimmt werden kann.

Die Aufgabe wird gelöst durch die Merkmale des Anspruchs 1.- Weitere Besonderheiten der Erfindung ergeben sich aus den Unteransprüchen.

Die GDP-Mannose-Pyrophosphorylase-Aktivität wurde nachgewiesen mit einem neu entwickelten kontinuierlichen spektrophotometrischen Test zur Bestimmung von Pyrophosphat ($PP_i$) produzierenden Nucleotidyltransferasen (EC 2.7) . In der im folgenden beschriebenen Weise kann durch Vorgabe der Substrate (Zucker-1-Phosphate bzw. Zucker im Falle der Neuraminsäure) und Nucleosidtriphosphate) jede beliebige Pyrophosphorylase-Aktivität, die $\geq 0{,}2$ mU/ml Aktivität im Testgemisch ausmachen kann, bestimmt werden.

Der Enzymtest (Nucleotidyltransferase substrate screening assay 'NUSSA') beruht darauf, daß das bei der Nucleotidyltransferase-Reaktion (EC 2.7-7) entstehende Pyrophosphat mit einer Pyrophosphat-abhängigen Phosphofuctokinase ($PP_i$PFK aus Pflanzen oder Bakterien EC 2.7.1.90) mit Fruktose-6-Phosphat, in Gegenwart von Fructose-2,6-Bisphosphat, zu Fructose-1,6-Bisphosphat umgesetzt wird. Dieses Produkt wird mit einer Aldolase zu Dihydroxyacetonphosphat (DHAP) und Glycerin-3-Phosphat (GAP) gespalten. Aus Glycerinaldehyd-3-Phosphat entsteht Dihydroxyacetonphosphat durch die Triosephosphatisomerase. Schließlich wird Dihydroxyacetonphosphat durch die Glycerin-3-Phosphat-Dehydrogenase zu Glycerin-3-Phosphat (G-3-P) mit NADH reduziert. Pro Mol Pyrophosphat werden 2 Mol NADH verbraucht, was photometrisch verfolgt werden kann.

| Reaktionsschema 2: | |
| --- | --- |
| GTP + Mannose-1-Phosphat | GDP-Mannose + $PP_i$ (1) |
| $PP_i$ + Fructose-6-Phosphat | Fructose-1,6-$P_2$ + $P_i$ (2) |
| Fructose-1,6-$P_2$ | DHAP + GAP (3) |
| DHAP + GAP | 2 GAP (4) |
| 2 GAP + 2 NADH | 2 G-3-P + 2 NAD (5) |
| (1) GDP-Mannose-Pyrophosphorylase<br>(2) Pyrophosphat-abhängige Phosphofructokinase<br>(3) Aldolase<br>(4) Triosephosphat-Isomerase<br>(5) Glycerin-3-Phosphat-Dehydrogenase | |

Die nachfolgenden Beispiele zeigen die erfindungsgemäße Arbeitsweise im einzelnen.

Beispiel

Nucleotidyltransferase substrate screening assay (NUSSA)

| Reaktionsschema 2 | | | |
| --- | --- | --- | --- |
| | (1) | NTP + Zucker-I-Phosphat | NDP-Zucker + $PP_i$ |
| | (2) | PPi + Fructose-6-Phosphat | Fructose-1,6-$P_2$ + $P_i$ |
| | (3) | Fructose-1,6-Pi | DHAP + GAP |
| | (4) | DHAP + GAP | 2 D HAP |
| | (5) | 2 DHA.P + 2 NADH + $H^+$ | 2 G-3-P + $NAD^+$ |
| (1) Pyrophosphorylase, (2) pyrophosphatabhängige Phosphofructokinase, (3) Aldolase, (4) Triosephosphat-Isomerase, (5) Glycerin-3-Phosphat-Dehydrogcnase NTP: Nucleosidtriphosphat / NDP: Nucleosiddiphosphat / DHAP: Dihydroxyacetonphosphat / GAP: Glycerinaldehyd-3-Phosphat / G-3-P: Glycerin-3-Phosphat / NAD: Nicotinsäureamidadenosindiphosphat, reduzierte Form | | | |

O'Brien, Bowien und Wood beschrieben 1975 in J. Biol. Chem. 250 (22), 8690-8695 einen gekoppelten photometrischen Enzymtest zur Messuna von einer pyrophosphatabhängigen Phosphofructokinase ($PP_i$PFK), wie sie erstmalig von Reeves et al., 1974 in J. Biol. Chem. 249, 7737-7741, in Entamoeba histolytica entdeckt wurde. Hierbei wurde die $PP_i$PFK gekoppelt mit der Reaktion der Aldolase (Reaktion 3), der Triosephosphatisomerase (Reaktion 4) und der Glycerin-3-Phosphat-Dehydrogenase (Reaktion 5). Die Reaktion wurde photometrisch bei 340 nm verfolgt.

Der folgende Enzymtest (NUSSA) koppelt erfindungsgemäß die Reaktion der Nucleotidyltransferase mit diesem Testsystem und ermöglicht somit die Messung einer beliebigen Pyrophosphorylase bzw. eines beliebigen Pyrophosphat-freisetzenden Enzyms- Der Test NUSSA wurde optimiert auf die Messung in Microtiterplatten in 200 µl Gesamtvolumen.

Tabelle 6

| Zusammensetzung des Enzymtests NUSSA | | | |
|---|---|---|---|
| | Endkonzentration | $PP_i$PFK-Test | Pyrophosphorylase-Test |
| Tris-HCl, pH 8 | 50 mM | | |
| MgCl$_2$*6 H$_2$O | 5 mM | 128 µl | 108 µ ± x µl |
| NADH | 0,15 mM | 10 µl | 10 µl |
| Fructose-6-Phosphat | 2,5 mM | 10 µl | 10 µl |
| Fructose-2,6-P$_2$ | 1 µM | 10 µl | 10 µl |
| PP$_i$ | 2,5 mM | 10 µl | --- |
| Zucker-1-Phosphat | variabel | --- | 10 µl |
| Nukleosidtriphosphat | variabel | --- | 10 µl |
| PP$_i$PFK-Präparation | variabel | 20 µl | 10 µl ± x µl |
| Aldolase | 0,09 U/200 µl | 4 µl | 4 µl |
| Triosephosphat-Isomerase | 1 U/200 µl | 4 µl | 4 µl |
| Glycerin-3-Phosphat-Dehydrogenase | 0,136 U/200 µl | 4 µl | 4 µl |
| Pyrophosphorylase-Präparation | variabel | --- | 20 µl |

Das Gesamtvolumen betrug 200 µl. Die Ansätze wurden in einem Titertek-Photometer Molecular Devices, München) photometrisch vermessen. In Falle des $PP_i$PFK-Tests wurde mit $PP_i$ gestartet, im Falle der Pyrophosohorylasen wahlweise mit dem Zuckerl-Phosphat oder dem Nucleosidtriphosphat.

Zur Berechnung der Aktivität wurde folgende Formel angewendet:

$$U/ml = \frac{\Delta E \text{ (m0D/Zeit)} * \text{Probenverdünnung} * \text{Meßvolumen}}{1000 * \text{Probenvolurnen} * d * \varepsilon_{NADH} * 2}$$

$$= \frac{10^{-3}\ \Delta E/min * \text{Probenverdünnung} * 200\ \mu l}{10^3 * 20\mu l * 0,67\ cm * 6,3(1*mmol\ l^{-1}\ *cm^{-1}) * 2}$$

$$= \Delta E * \text{Probenverdünnung} * 0,0012\ \mu mol/ml * min$$

Anwendung des Enzymtests NUSSA

1) Beispiel siehe oben: Aktivitätsmessungen der GDP-Mannose-Pyrophosphorylase

2) Beispiel : Anwendung des NUSSA zum Screening auf Pyrophosidhorylasen in zwei verschiedenen Enzymquellen:

a) Escherichia coli BL21(DE3)pLysSpERJ-1
b) Reis (Oryza sativa L.)

Zur Anwendung des Tests muß die $PP_i$PFK aufgereinigt werden. Als einfache und gut verfügbare Enzymquelle wurde die Kartoffel (Solanum tuberosum L.) gewählt. Die Aufreinigung wurde nach der von van Schaftingen et al., 1982 in Eur. J. Biochem. 129, 191-195 beschriebenen Methode durchgeführt. Das Enzym (PP$_i$PFK) wurde in 25% Glycerin bei -20°C aufbewahrt.

Escherichia coli BL21(DE3)pLysSpERJ-1 wurde, wie oben beschrieben, angezogen und aufgeschlossen. Das erhaltene Rohhomogenat wurde bei 10000 rpm, 2 min, 20°C abzentrifugiert und im Enzymtest eingesetzt (21.02 mg/ml). Der Reis wurde nach Elling, 1993 in Patent DE 42 21 595 C1, aufgeschlossen, bei- 10000 rpm, 10 min, 20 OC abzentrifugiert und als Rohextrakt (4.26 mg/ml) im Enzymscreening eingesetzt. Als Substrate wurden getestet:

a) Nucleosidtriphosphate: ATP, CTP, GTP, UTP, dTTP je 1 mM im Test mit Glucose-1-Phosphat (2.5 mM)

b) Nucleosidtriphosphat UTP (1 mM) Zucker-1-Phosphate je 2,5 mM im Test

Die Tabelle 7 zeigt die spezifischen Aktivitäten von Pyrophosphorylasen in einer mikrobiellen und einer eukaryotischen Enzymquelle.

Tabelle 7

| Spezifische Aktivitäten von Pyrophosphorylasen in E.coli und Reis | | | |
|---|---|---|---|
| α-D-Zucker-1-Phosphate [2,5 mM] | NTPs [ 1 mM] | *E. coli* [mU/mg] | Reis [mU/mg] |
| α-D-Glucose-1-Phosphat | + ATP | 2,76 | - |
| | + CTP | 0,75 | - |
| | + GTP | - | - |
| | + UTP | 214,10 | 982,40 |
| | + dTTP | 10,32 | 5,98 |
| α-D-Glucosamin-1-P | + UTP | 2,75 | - |
| α-D-GlcNAc-1 | + UTP | 2,02 | 0,27 |
| α-D-Galactose-1-P | + UTP | 1 | 10,41 |
| α-D-Galactosamin-1-P | + UTP | - | 0,25 |
| α-D-GalNAc-1-P | + UTP | - | 0,18 |
| α-D-Glucuronsäure-1-P | + UTP | 4,72 | 14,27 |
| α-D-Galacruronsäure-1-P | + UTP | 3.66 | 2,94 |
| α-D-Xylose-1-P | + UTP | - | *0,42* |
| α-D-Manose-1-P | + GTP | 3,41 | 1,51 |
| 20 μg/ml E. coli BL21(DE3)pLysS-pJER-1-Rohextrakt im Testansatz, 1,06 μg/ml bis 0,14 mg/ml Oryza sativa-Rohextrakt im Testansatz. Aktivitätsbestimung per NUSSA | | | |

## Patentansprüche

1. Photometrischer Test, bei dem das durch ein Pyrophosphat freisetzendes Enzym entstehende Pyrophosphat mittels einer Pyrophosphat-abhänigigen Phosphofruktokinase, einer Aldolase, einer Triosephosphat-Isomerase und einer Glycerin-3-Phosphat-Dehydrogenase umgesetzt und die durch die Dehydrogenase erfolgende Reduktion photometrisch bestimmt wird.

2. Photometrischer Test nach Anspruch 1 zur Bestimmung von Pyrophosphat freisetzenden Nucleotidyltransferasen.

3. Photometrischer Test nach Anspruch 2 zur Bestimmung der GDP-Mannose-Pyrophosporylase.

## Claims

1. A photometric test in which the pyrophosphate formed by an enzyme which releases pyrophosphate is converted by means of a pyrophosphate-dependent phosphofructokinase, an aldolase, a triose phosphate isomerase and a glycerin-3-phosphate dehydrogenase and the reduction which is effected by the dehydrogenase is determined photometrically.

2. A photometric test according to claim 1 for the determination of nucleotidyl transferases which release pyrophosphate.

3. A photometric test according to claim 1 for the determination of GDP mannose pyrophosphorylase.

**Revendications**

1. Test photométrique dans lequel on fait réagir le pyrophosphate se formant par un enzyme dégageant du pyrophosphate au moyen d'une phosphofructokinase dépendant du pyrophosphate, d'une aldolase, d'une triosephosphate-isomérase et d'une glycérine-3-phosphate-deshydrogénase et l'on détermine photométriquement la réduction s'effectuant par la deshydrogénase.

2. Test photométrique suivant la revendication 1, de détermination de nucléotidyltransférases dégageant du pyrophosphate.

3. Test photométrique suivant la revendication 2, de détermination de la GDP-mannose-pyrophosporylase